# EUROPÄISCHE PATENTSCHRIFT

(11) **EP 2 260 003 B1**
(45) Veröffentlichungstag und Bekanntmachung des Hinweises auf die Patenterteilung: **29.06.2016**
(21) Anmeldenummer: 09727160.5
(22) Anmeldetag: 03.04.2009
(51) Int. Cl.: C01G 39/00, C01G 53/00, C07C 51/16, B01J 23/887, B01J 27/192, C01G 41/00, B01J 23/888

(54) **VERFAHREN ZUR HERSTELLUNG EINES NANOKRISTALLINEN MOLYBDÄNMISCHOXIDS**
METHOD FOR PRODUCING A NANOCRYSTALLINE MOLYBDENUM MIXED OXIDE
PROCÉDÉ POUR PRODUIRE UN OXYDE MIXTE NANOCRISTALLIN À BASE DE MOLYBDÈNE

(30) Priorität: 04.04.2008 DE 102008017311
(43) Veröffentlichungstag der Anmeldung: 15.12.2010
(73) Patentinhaber: Süd-Chemie IP GmbH & Co. KG, 80333 München (DE)
(72) Erfinder: HAGEMEYER, Alfred, 83043 Bad Aibling (DE); MESTL, Gerhard, 80935 München (DE); NEUMANN, Silvia, 83109 Grosskarolinenfeld (DE); WÖLK, Hans-Jörg, 83022 Rosenheim (DE)
(74) Vertreter: Silber, Anton
(86) Internationale Anmeldenummer: PCT/EP2009/002476
(87) Internationale Veröffentlichungsnummer: WO 2009/121626

(56) Entgegenhaltungen:
- WO-A-2004/024666
- DE-A1-102006 032 452
- FR-A- 2 826 959
- GB-A- 1 538 107
- US-A1- 2008 161 602

## Beschreibung

Die Erfindung betrifft ein Verfahren zur Herstellung eines nanokristallinen Molybdänmischoxids, die Verwendung des Molybdänmischoxids als Katalysator für chemische Umsetzungen sowie einen Katalysator, der das Molybdänmischoxid enthält.

Molybdänmischoxide werden bisher im Stand der Technik durch Fällungsmethoden, Sol-Gel-Verfahren oder Festkörperreaktionen erhalten.

Molybdänmischoxide werden im Stand der Technik als Katalysator für chemische Umsetzungen eingesetzt. Beispielhaft genannt seien hier Umsetzungen von Alkylverbindungen oder Alkenverbindungen zu Acrolein oder dessen Derivaten sowie eine Umsetzung von Acrolein zu Acrylsäure. Häufig zeigen die Molybdänmischoxidkatalysatoren gemäß dem Stand der Technik keine ausreichende Aktivität in diesen Reaktionen.

Die WO 2008/028681 und WO 2008/006565 A1 offenbaren ein Verfahren zur Herstellung nanokristalliner Metalloxide oder Metall-Mischoxide. In diesen Schriften findet sich kein Hinweis, dass mit den Verfahren spezielle nanokristalline Molybdänmischoxide hergestellt werden können, die sich als Katalysator insbesondere für die Umsetzung von Acrolein zu Acrylsäure besonders gut eignen.

Über herkömmliche Verfahren lässt sich ein kristallines Molybdänmischoxid nur schwer erhalten. So offenbart G.A. Zenkovets et. al., "The structural genesis of a complex (MoVW)5O14 oxide during thermal treatments and its redox behaviour at elevated temperatures", Materials Chemistry and Physics, 103 (2007), 295-304, dass ein über Sprühtrocknung erhaltenes Mo_{0,68}V_{0,23}W_{0,09}Oₓ-Mischoxid eine amorphe Struktur aufweist. Dieses Mischoxid liegt in Form von großen Aggregaten von etwa 5 µm Größe vor. Durch nachfolgendes Kalzinieren bildet sich innerhalb der Aggregate eine teilweise nanokristalline Struktur aus. Erst nach längerer thermischer Behandlung bei etwa 440 °C bildet sich eine reine kristalline Phase mit Kristalliten von mehr als 1000 nm Größe. Die Herstellung eines nanokristallinen Molybdänmischoxids lässt sich also nur schwer bewerkstelligen.

Aus O. Ovsiter et. al., "Molybdenum oxide based partial oxidation catalyst Part 3", Journal of Molecular Catalysis A: Chemical 185 (2002), 291-303 geht hervor, dass ein Molybdänmischoxid nach thermischer Behandlung keine gut kristallisierten Partikel zeigt. Eine kristalline Phase konnte jedoch während einer Umsetzung von Acrolein zu Acrylsäure beobachtet werden, d.h., eine Kristallisation erfolgt erst bei der Oxidationsreaktion. Ein solcher Katalysator weist dadurch erst nach längerer Reaktionsdauer eine ausreichende Aktivität auf.

Nachteilig an den im Stand der Technik beschriebenen Molybdänmischoxiden ist somit, dass keine einheitliche Partikelgröße der Molybdänmischoxide erhalten werden kann und eine Steuerung der Kristallisation, insbesondere hinsichtlich der Kristallitgröße, nicht möglich ist. Ebenso ist die BET-Oberfläche der im Stand der Technik beschriebenen Molybdänmischoxide meist zu gering. Gewünscht, insbesondere für katalytische Anwendungen, ist eine kleine Partikelgröße mit möglichst großer BET-Oberfläche.

Aufgabe der vorliegenden Erfindung war somit die Bereitstellung eines nanokristallinen Molybdänmischoxids, das bei katalytischen Umsetzungen, insbesondere bei einer Umsetzung von Acrolein zu Acrylsäure, eine erhöhte Aktivität und Selektivität aufweist.

Die Aufgabe wird gelöst durch ein Verfahren gemäß Anspruch 1.

Überraschenderweise wurde gefunden, dass durch das erfindungsgemäße Verfahren eine einheitliche Partikelgröße der Molybdänmischoxide erhalten werden kann und eine Steuerung der Kristallisation, insbesondere hinsichtlich der Kristallitgröße, erreicht wird. Ebenso konnte die BET-Oberfläche gegenüber den im Stand der Technik bekannten Molybdänmischoxiden erhöht werden.

Das erfindungsgemäß erhaltene Molybdänmischoxid zeichnet sich durch eine Kristallitgröße im Bereich von 10 nm bis 450 nm aus.

Die katalytische Aktivität eines Katalysators enthaltend das erfindungsgemäß erhaltene Molybdänmischoxid, insbesondere bei einer Umsetzung von Acrolein zu Acrylsäure, konnte um etwa 1 % verglichen mit herkömmlichen bekannten Katalysatoren gesteigert werden.

Unter dem Begriff "Mischoxid" ist im Sinne der Erfindung ein gemischtes Oxid zu verstehen, das zwei oder mehr Metalle umfasst und eine einzige chemische Verbindung darstellt, die mit einer Formel ausgedrückt werden kann. Zu unterscheiden ist dies entsprechend von einer reinen (physikalischen) Mischung aus mehreren Metalloxiden.

Als Molybdänausgangsverbindung wird ein Molybdat, besonders bevorzugt Ammoniumheptamolybdat-tetrahydrat verwendet. Jedoch ist dem Fachmann auf diesem Gebiet klar, dass auch andere im Stand der Technik bekannte Molybdate und Molybdänverbindungen verwendet werden können.

Um ein Mischoxid zu erhalten, sollte wenigstens eine weitere metallhaltige Ausgangsverbindung verwendet werden. Verbindungen gemäß der vorliegenden Erfindung sind Wolframate und/oder Vanadate. Besonders bevorzugt ist die weitere Ausgangsverbindung Ammoniummetawolframat und/oder Ammoniummetavanadat. Am meisten bevorzugt ist eine Kombination der beiden zuletzt genannten.

Die Molybdänausgangsverbindung und die wenigstens eine weitere metallhaltige Ausgangsverbindungen werden zusammen als Lösung, Suspension oder Aufschlämmung eingesetzt.

Am meisten ist es bevorzugt, wenn die Ausgangsverbindungen in Lösung, insbesondere in wässriger Lösung, vorliegen. Falls nötig kann die Lösung erwärmt werden, um eine vollständige Auflösung der Ausgangsverbindungen zu erreichen, insbesondere bei schlecht löslichen Ausgangsverbindungen. Vorteilhaft wird die Lösung der Ausgangsverbindungen auf > 50 °C erwärmt.

In einer besonders bevorzugten Ausführungsform wird in dem erfindungsgemäßen Verfahren eine Lösung aus Ammoniummetawolframat, Ammoniumheptamolybdat-tetrahydrat und Ammoniummetavanadat verwendet.

Zu der Lösung, Suspension oder Aufschlämmung, welche die Molybdänausgangsverbindung und die wenigstens eine weitere metallhaltige Ausgangsverbindung enthält, wird vor dem oder während dem Einbringen in die Reaktionskammer eine Lösung, Suspension oder Aufschlämmung, welche wenigstens ein zusätzliches Metallsalz enthält, gegeben. Das wenigstens eine Metallsalz ist ein Kupfersalz oder ein Eisensalz oder eine Kombination eines Kupfer- und eines Eisensalzes.

Die Zugabe einer Metallsalz enthaltenden Lösung, Suspension oder Aufschlämmung zu der Lösung, Suspension oder Aufschlämmung, welche die Molybdänausgangsverbindung und die wenigstens eine weitere metallhaltige Ausgangsverbindung enthält, bewirkt eine Dotierung des resultierenden Molybdänmischoxids mit dem entsprechenden Metall. Dadurch können beliebige Feinabstimmungen für die jeweilige gewünschte katalytische Funktion des Molybdänmischoxids erreicht werden.

In einer besonders bevorzugten Ausführungsform wird eine Lösung aus Ammoniummetawolframat, Ammoniumheptamolybdat-tetrahydrat und Ammoniummetavanadat, zu der eine Kupfersalzlösung gegeben wird, in dem erfindungsgemäßen Verfahren verwendet. Bevorzugt ist das Kupfersalz Kupfersulfat oder Kupferacetat.

In einer weiteren besonders bevorzugten Ausführungsform wird eine Lösung aus Ammoniummetawolframat, Ammoniumheptamolybdat-tetrahydrat und Ammoniummetavanadat, zu der eine Kupfersalz- und Eisensalzlösung gegeben wird, in dem erfindungsgemäßen Verfahren verwendet. Bevorzugt ist das Kupfersalz Kupfersulfat oder Kupferacetat und das Eisensalz ist bevorzugt Eisennitrat.

Überraschenderweise wurde gefunden, dass das Verfahren bei relativ niedrigen Temperaturen von 200 bis 500 °C, besonders bevorzugt von 250 bis 450 °C, besonders bevorzugt von 300 bis 400 °C durchgeführt werden kann. Bislang waren im Stand der Technik bevorzugte Temperaturen von mehr als 700 °C, und zwar bis zu 1400 °C bekannt. Ganz besonders überraschend wurde auch gefunden, dass durch das erfindungsgemäße Verfahren der Kristallisationsprozess des Molybdänmischoxids gezielt gesteuert werden kann, insbesondere die Größe der Kristallite und die Porengrößenverteilung der entsprechenden Molybdänmischoxide. Dies kann weiter durch die Verweilzeit in der Flamme bzw. durch die Reaktortemperatur vorteilhaft beeinflusst werden. Durch die pulsierende thermische Behandlung werden die entstehenden nanokristallinen Molybdänmischoxidpartikel daran gehindert zu agglomerieren. Typischerweise werden die nanokristallinen Partikel sofort durch den Strom an heißem Gas in eine kältere Zone überführt, wo die Molybdänmischoxidkristallite zum Teil sogar mit Durchmessern von weniger als 20 nm erhalten werden.

Dies führt bei den so erhältlichen Molybdänmischoxidkristalliten zu deutlich erhöhten BET-Oberflächen von > 1 m²/g, besonders bevorzugt 2 bis 10 m²/g und besonders bevorzugt 3 bis 7 m²/g. Die Bestimmung der BET-Oberfläche erfolgt nach der Methode von Brunauer, Emmett und Teller gemäß DIN 66132.

In dem erfindungsgemäßen Verfahren können Suspensionen ohne zusätzliche Filtrations- und/oder Trocknungsschritte bzw. ohne Zugabe von zusätzlichen Lösungsmitteln innerhalb eines sehr kurzen Zeitraumes, typischerweise innerhalb weniger Millisekunden, bei vergleichsweise niedrigeren Temperaturen als bei Verfahren des Standes der Technik üblich, kalziniert werden. Die entstehenden Molybdänmischoxid-Nanokristallite weisen signifikant erhöhte BET-Oberflächen auf und stellen somit einen Molybdänmischoxidkatalysator mit erhöhter Reaktivität, verbessertem Umsatz und verbesserter Selektivität, insbesondere hinsichtlich einer Umsetzung von Acrolein zu Acrylsäure, dar.

Durch die annähernd gleiche Verweilzeit jedes Molybdänmischoxidpartikels in dem durch das Verfahren erzeugten homogenen Temperaturfeld entsteht ein äußerst homogenes Endprodukt mit enger monomodaler Teilchenverteilung. Eine Vorrichtung zur Durchführung des erfindungsgemäßen Verfahrens bei der Herstellung derartiger monomodaler nanokristalliner Metalloxidpulver ist beispielsweise aus der DE 101 09 892 A1 bekannt. Im Gegensatz zu der dort beschriebenen Vorrichtung und dem dort offenbarten Verfahren benötigt das vorliegende Verfahren jedoch keinen vorgelagerten Verdampfungsschritt, in dem der Ausgangsstoff, d.h. die Molybdänausgangsverbindung, auf eine Verdampfungstemperatur erwärmt wird.

Die Molybdänausgangsverbindung und die weiteren Ausgangsverbindungen, aus der die erfindungsgemäßen Molybdänmischoxide hergestellt werden, werden direkt über ein Trägerfluid, insbesondere ein Trägergas, vorzugsweise ein inertes Trägergas, wie beispielsweise Stickstoff, in so genannte Reaktionskammern, d.h. in die Brennkammer, eingeführt. An die Reaktionskammer ist abgasseitig ein Resonanzrohr mit einem gegenüber der Reaktionskammer deutlich verringertem Strömungsquerschnitt angeschlossen. Der Brennkammerboden ist mit mehreren Ventilen zum Eintritt der Verbrennungsluft in die Brennkammer ausgestattet. Die aerodynamischen Ventile sind dabei strömungstechnisch und akustisch so mit der Brennkammer und der Resonanzrohrgeometrie abgestimmt, dass die in der Brennkammer erzeugten Druckwellen des homogenen "flammenlosen" Temperaturfeldes sich vorwiegend im Resonanzrohr pulsierend ausbreiten. Es bildet sich ein so genannter Helmholtzresonator mit pulsierender Strömung aus mit einer Pulsationsfrequenz zwischen 10 und 150 Hz, bevorzugt 30 bis 110 Hz.

Die Materialzuführung in die Reaktionskammer erfolgt typischerweise entweder mit einem Injektor oder mit einer geeigneten Zweistoffdüse oder in einem Schenkdosierer.

Bevorzugt wird die Molybdänausgangsverbindung in verdüster Form in die Reaktionskammer eingebracht, so dass eine feine Verteilung im Bereich der Behandlungszonen gewährleistet ist. Nach der thermischen Behandlung werden die entstandenen nanokristallinen Molybdänmischoxide, wenn möglich mittels des Trägerfluids, sofort in eine kältere Zone der Reaktionskammer überführt, sodass sie in der kälteren Zone abgeschieden und ausgetragen werden können. Die Ausbeute des erfindungsgemäßen Verfahrens beträgt nahezu 100%, da das entstehende Produkt vollständig aus dem Reaktor ausgetragen werden kann.

Typischerweise wird das Verfahren bei einem Druck zwischen 15 und 40 bar durchgeführt.

Gegenstand der Erfindung ist ferner das durch das erfindungsgemäße Verfahren erhältliche nanokristalline Molybdänmischoxid. Es wurde gefunden, dass das so erhältliche nanokristalline Molybdänoxid eine Kristallitgröße im Bereich von 10 nm bis 450 nm, bevorzugt von 10 nm bis 400 nm, ganz besonders bevorzugt 15 bis 250 nm aufweist, was, wie vorstehend schon ausgeführt, durch die Pulsation der thermischen Behandlung bevorzugt eingestellt werden kann. Die Bestimmung der Kristallitgröße kann durch XRD oder TEM erfolgen.

Weiterhin werden durch das erfindungsgemäße Verfahren Molybdänoxidpartikel erhalten, die eine BET-Oberfläche von vorzugsweise > 1 m²/g, besonders bevorzugt 2 bis 10 m²/g und besonders bevorzugt 3 bis 7 m²/g besitzen.

Das erfindungsgemäß erhaltene Molybdänmischoxid eignet sich in hervorragender Weise zur Verwendung als Katalysator, beispielsweise bei der katalytischen Umsetzung von Acrolein zu Acrylsäure.

Acrylsäure oder Propensäure gehört zu den ungesättigten Carbonsäuren. Acrylsäure ist eine farblose, mit Wasser mischbare bei Raumtemperatur flüssige chemische Verbindung mit stechendem, essigähnlichem Geruch. Acrylsäure wirkt stark korrodierend und ist entzündlich. Die großindustrielle Herstellung erfolgt gewöhnlich durch eine zweistufige Oxidation von Propen mit Hilfe von Katalysatoren. In der ersten Stufe wird Propen mit Luft zu Propenal (Acrolein) umgesetzt. In der zweiten Stufe erfolgt die Oxidation von Propenal zu Acrylsäure. Ihre Hauptverwendung ist die Polymerisation zu Superabsorbern (Anwendung z. B. in Windeln), Acrylatestern (die wiederum zur Herstellung von Polymeren verwendet werden) und als Comonomer bei der Herstellung von Polymerdispersionen. Die wasserlöslichen Polymerisate der Acrylsäure werden als Appreturen und Verdickungsmittel sowie als Überzüge für feste Arzneiformen und als Salbengrundlagen verwendet. Der Polyacrylsäureethylester hat sich als Copolymerisationspartner zur Herstellung witterungsbeständiger Elastomere bewährt.

Gegenstand der Erfindung ist somit auch ein Katalysator, der das erfindungsgemäße Molybdänmischoxid enthält. Der Katalysator kann ein geträgerter oder ein ungeträgerter Katalysator (Vollkatalysator, Extrudatkatalysator) sein.

Das Molybdänmischoxid kann in einer Ausführungsform der vorliegenden Erfindung zusammen mit einem geeigneten Bindemittel zu einem Extrudat (Tabletten, Formkörper, Honigwabenkörper und dergleichen) verarbeitet werden. Als Bindemittel kann jedes dem Fachmann geläufige und geeignet erscheinende Bindemittel verwendet werden, insbesondere Silicatmaterialien, Aluminiumoxid, Zirkoniumverbindungen, Titanoxid, sowie deren Mischungen, und Materialien, wie z.B. Zement, Ton, Silika/Alumina. Bevorzugte Bindemittel sind unter anderem Pseudoboehmit sowie silikatische Bindemittel wie kolloidales Siliziumoxid oder Silikasol.

Das Molybdänmischoxid kann in bevorzugten Weiterbildungen der Erfindung ferner zusammen mit anderen Komponenten, vorzugsweise mit einem Bindemittel, besonders bevorzugt mit einem organischen Bindemittel, beispielsweise organische Kleber, Polymere, Harze oder Wachse, zu einem Washcoat verarbeitet werden, der auf einen metallischen oder keramischen Träger aufgebracht werden kann. Gegebenenfalls können zusätzliche Imprägnierschritte oder Kalzinierschritte erfolgen. Bevorzugt liegt das erfindungsgemäß erhaltene Molybdänmischoxid als Beschichtung auf einem Träger vor. Bevorzugtes Trägermaterial ist Steatit, besonders bevorzugt sind Steatitkugeln. Die Beschichtung wird bevorzugt in einer dem Fachmann an sich bekannten Wirbelbett-Coating-Vorrichtung durchgeführt.

Gegenstand der Erfindung ist auch ein Verfahren zur Umsetzung von Acrolein zu Acrylsäure, wobei ein oben definierter Katalysator verwendet wird.

In dem Verfahren wird Acrolein, vorzugsweise mit Sauerstoff, Wasserdampf und Stickstoff bei 200 bis 400 °C über ein Bett des erfindungsgemäßen Katalysators geleitet. Es zeigt sich eine Verbesserung in dem erfindungsgemäßen Verfahren hinsichtlich Acroleinumsatz, Acrylsäureselektivität und Acrylsäureausbeute gegenüber einem Verfahren, das mit einem Katalysator nach dem Stand der Technik durchgeführt wurde.

Die Erfindung wird anhand der nachstehenden und nicht als einschränkend zu verstehenden Ausführungsbeispiele und der Figuren näher erläutert. Die verwendete Vorrichtung entspricht dabei weitgehend der in der DE 101 09 892 A1 beschrieben Vorrichtung, mit dem Unterschied, dass die zur Durchführung des erfindungsgemäßen Verfahrens verwendete Vorrichtung keine Verdampfervorstufe aufwies.

Es zeigen:

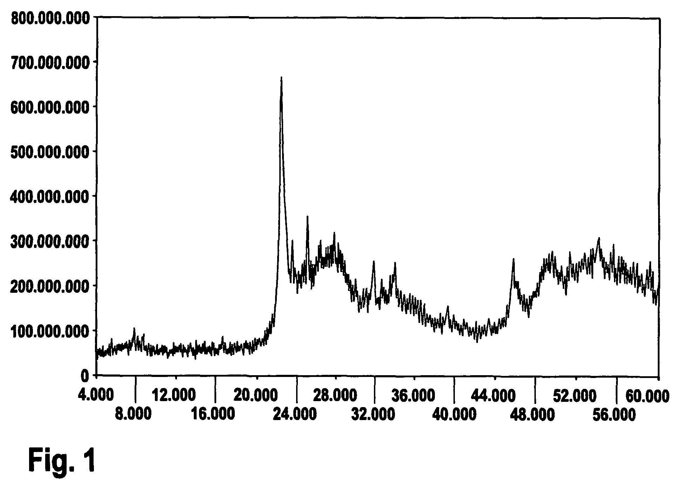

- Figur 1: das XRD Spektrum des in Beispiel 6 erhaltenen erfindungsgemäßen Molybdänmischoxids

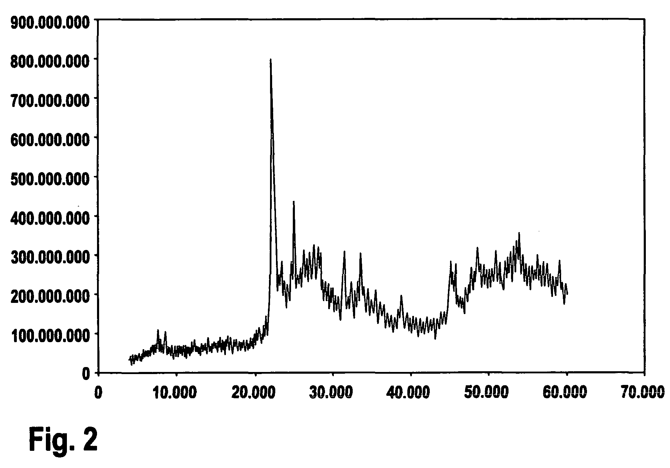

- Figur 2: das XRD Spektrum des in Beispiel 7 erhaltenen erfindungsgemäßen dotierten Mischoxids

### Ausführungsbeispiele:

### Allgemeines

Die wesentlichen Vorteile der Präparation mit Hilfe des Pulsationsreaktors liegen in der Verkürzung der gesamten Präparationszeit, des geringen Aufwandes (benötigt wird nur der Reaktor) und darin, dass Trocknung und Aufbereitung des Produktes entfallen. Durch den Pulsationsreaktor lassen sich in einem Schritt die gewünschten BET-Oberflächen, Partikelgrößen und auch die Kristallinität des Materials variieren.

Folgende Methoden wurden für die Präparation der Mischoxide variiert:

### Beispiel 1: MoWV Variante 1 (Referenzbeispiel)

Für die Herstellung wurden zwei Lösungen hergestellt.

Für Lösung 1 wurden 18 1 dest. H₂O in einem Stahlfass vorgelegt und erwärmt. 487,8 g Ammoniummetawolframat wurde zugegeben und gelöst. Nach Erreichen von 70 °C erfolgte die Zugabe von 3300 g Ammoniumheptamolybdat-tetrahydrat und 546,6 g Ammoniummetavandadat. Nach vollständigem Auflösen der Komponenten wurde 113,4 g Antimontrioxid zugegeben und 1 Stunde bei 95 °C gerührt. Die Lösung wurde auf Raumtemperatur abgekühlt.

Lösung 2 wurde durch Lösen von 466,8 g Kupfersulfat in 2880 ml dest. H₂O bei Raumtemperatur hergestellt. Die beiden Lösungen wurden bei Raumtemperatur vermischt und ca. 1h gerührt. Es entstand eine Suspension.

Die Verdüsung der Lösung wurde folgendermaßen im Pulsationsreaktor durchgeführt: nach apparativ bedingter Aufheizzeit von mehreren Stunden wurde die Suspension bei verschiedenen Reaktortemperaturen (380, 360, 400°C) versprüht.

Verwendet wurde Luft als Trägergas. Die BET-Oberflächen des erhaltenen Molybdänmischoxids lagen zwischen 1 bis 4 m²/g.

### Beispiel 2: MoWV Variante 2 (Referenzbeispiel)

Für die Herstellung wurde 11820 ml destilliertes H₂O vorgelegt. Dazu wurde 3150,12 g Ammoniumheptamolybdat-tetraydrat gegeben und gelöst. Während des gesamten Herstellungsprozesses wurde stetig gerührt.

Danach wurde 822,24 g Ammoniummetawolframat zugegeben und gelöst. Nach anschließendem Aufheizen auf ca. 80 °C wurde anschließend 585,6 g Ammoniummetavanadat zugegeben und weiter auf 100 °C erwärmt. Diese Temperatur wurde 5 Stunden gehalten, danach wurde die Lösung auf Raumtemperatur abgekühlt.

Abschließend wurde ebenfalls bei Raumtemperatur 444 g Ammoniumacetat zugegeben.

Die Hälfte des 5 kg-Ansatzes wurde mit Eisen und Kupfer dotiert.

Dazu wurden 1,86 g Kupferacetat und 4,14 g Eisennitrat in jeweils 120 ml dest. H₂O gelöst und anschließend in die Lösung gegeben.

Die Lösung wurde 12 h unter Rückfluss erhitzt. Dabei entstand eine orange-rot gefärbte klare Lösung.

Diese Lösung wurde in den Pulsationsreaktor unter Verwendung von Luft als Trägergas bei 380°C dotiert und undotiert verdüst. Es entstand ein grau gefärbtes Pulver mit BET-Oberflächen von 3-4 m²/g.

### Beispiel 3: MoWV Variante 3

11820 ml dest. H₂O wurden in einem Fass vorgelegt. In dieses wurde 3150,12 g Ammoniumheptamolybdat-tetrahydrat zugegeben, dann erhitzt und gewartet, bis es sich vollständig gelöst hatte.

Danach wurde 822,24 g Ammoniummetawolframat zugegeben und gelöst. Anschließend wurde auf ca. 80 °C aufgeheizt, dann 585,6 g Ammoniummetavanadat zugegeben und weiter auf 100 °C erwärmt. Diese Temperatur wurde 5 Stunden gehalten und dann auf Raumtemperatur abgekühlt.

Nach Abkühlung auf Raumtemperatur wurde 444 g Ammoniumacetat zugeben. Während des gesamten Herstellungsprozesses wurde stetig gerührt.

### Dotierung:

Der Ansatz wurde mit Eisen und Kupfer dotiert. Dazu wurden 3,72 g Kupferacetat und 8,28 g Eisennitrat in jeweils 240 ml dest. H₂O gelöst und anschließend in die Lösung gegeben.

### Verdüsung + Ergebnisse:

Die Lösung wurde an normaler Luft im Pulsationsreaktor verdüst.

### Lösung mit Dotierung:

| | | | |
|---|---|---|---|
| Temperatur: | 380°C | | |
| Farbe des Pulvers: | grau/schwarz | | |
| BET: | 3,2 | | |
| XRD: | Zeigt Hauptpeak bei 22 | | |
| Kristallitgröße: | 210 Å | | |
| Zusammensetzung: | W 14,7 % | Soll: | 9,3 % |
| | V 6,6 % | | 6,2 % |
| | Mo 45,9 % | | 46,9 % |
| | Fe < 220 ppm | | 2,5 % |
| | Cu 0,15 % | | 3,1 % |
| | | | |
| Pulver aus den Filtersäcken | | | |
| Temperatur: | 380 °C | | |
| Pulver: | grau/schwarz | | |
| BET: | 3,3 | | |
| XRD: | Zeigt Hauptpeak bei 22 | | |
| Kristallitgröße: | 201 Å | | |
| Zusammensetzung: | W 14,6 % | | |
| | V 6,7 % | | |
| | Mo 46,9 % | | |
| | Fe 240 ppm | | |
| | Cu 2,2 % | | |

### Beispiel 4: MoWV Variante 4

Lösung 1: 18 Liter dest. H₂O wurden in einem Stahlfass vorgelegt und erwärmt. 487,8 g Ammoniummetawolframat wurde zugegeben und gelöst. Bei Erreichen einer Temperatur ca. 70 °C wurden 3300 g Ammoniumheptamolybdat-tetraydrat und 546,6 g Ammonium-metavandadat zugegeben. Nach vollständigem Auflösen der Komponenten wurde 113,4 g Antimontrioxid zugegeben und 1 Stunde bei 95 °C gerührt. Anschließend wurde auf Raumtemperatur abgekühlt. Während des gesamten Herstellungsprozesses wurde stetig gerührt.

Lösung 2: 466,8 g Kupfersulfat wurden in 2880 ml dest. H₂O bei Raumtemperatur gelöst. Nach Abkühlung von Lösung 1 wurden die beiden Lösungen gemischt und 1 Stunde lang gerührt. Es entstand eine schwarze Lösung mit feinen weißen Partikeln.

### Verdüsung + Ergebnisse:

Alle Verdüsungen wurden mit normaler Luft im Pulsationsreaktor durchgeführt.

| | | | |
|---|---|---|---|
| Pulver aus dem Fass | | | |
| Temperatur: | 380°C | | |
| Pulver: | grau/schwarz | | |
| BET: | 1,0 m²/g | | |
| XRD: | Zeigt Hauptpeak bei 22 | | |
| Kristallitgröße: | 207 Å | | |
| Zusammensetzung | W 9,1 % | Soll: | 9,3 % |
| | V 6,0 % | | 6,2 % |
| | Mo 45,8 % | | 46,9 % |
| | Sb 1,8 % | | 2,5 % |
| | Cu 2,2 % | | 3,1 % |
| | | | |
| Pulver aus den Filtersäcken | | | |
| Temperatur: | 380°C | | |
| Pulver: | grau/schwarz | | |
| BET: | 1,5 | | |
| XRD: | Zeigt Hauptpeak bei 22 | | |
| Kristallitgröße: | 197 Å | | |
| Zusammensetzung: | W 9,2 % | Soll: | 9,3 % |
| | V 6,1 % | | 6,2 % |
| | Mo 46,5 % | | 46,9 % |
| | Sb 1,8 % | | 2,5 % |
| | Cu 2,2 % | | 3,1 % |

### Beispiel 5 (Vergleichsbeispiel)

Beispiel 5 wurde gemäß dem US 6,124,499 durchgeführt:

127 g Kupfer(II)acetat Monohydrat (Cu Gehalt 32,3 Gew.-%) wurden in 2700 g Wasser gelöst (Lösung I). 860g Ammoniumheptamolybdat-tetrahydrat (81,3 Gew.-% MoO₃), 143 g Ammoniummetavanadat (72,2 Gew.-% V₂O₅) und 126 g Ammoniumparawolframat-heptahydrat (89,3 Gew.-% WO₃) wurden der Reihe nach in 5500 g Wasser bei 95 °C (Lösung II) gelöst. Lösung I wurde dann auf einmal in Lösung II eingerührt und die wässrige Mischung sprühgetrocknet bei einer Ausgangstemperatur von 110 °C. Das erhaltene Sprühgut wurde mit 0,15 kg Wasser pro kg Pulver verknetet.

Die Knetmasse wurde in einem Umluftofen in einer Sauerstoff/Stickstoff Mischung kalziniert. Der Sauerstoffgehalt wurde so eingestellt dass die O₂-Ausgangskonzentration 1,5 Vol.-% am Ofenausgang betrug. Das Knetmaterial wurde zuerst mit einer Rate von 10 °C/min auf 300 °C aufgeheizt und dann bei 300 °C für 6 h gehalten. Anschließend wurde mit einer Heizrate von 10 °C/min auf 400 °C aufgeheizt und für 1 h bei 400 °C gehalten. Der Katalysator hatte die Zusammensetzung Mo₁₂V₃W_{1.2}Cu_{1.6}Oₓ.

Die kalzinierte Aktivmasse wurde auf 0.1 µm bis 50 µm herunter gemahlen

| | |
|---|---|
| Pulver: | grau/schwarz |
| BET: | 1,0 m²/g |
| XRD: | Zeigt Hauptpeak bei 22 |
| Kristallitgröße: | > 960 Å |

### Beispiel 6 (erfindungsgemäß)

### Lösung 1:

18 Liter dest. H₂O werden in einem Stahlfass vorgelegt und erwärmt. 487,8 g Ammoniummetawolframat wurde zugeben und gelöst. Als die Temperatur ca. 70 °C erreicht hatte, erfolgte eine Zugabe von 3300 g Ammoniumheptamolybdat-tetrahydrat und 546,6 g Ammoniummetavandadat. Nach vollständigem Auflösen der Komponenten wurde 113,4 g Antimontrioxid zugegeben und 1 h bei 95 °C gerührt. Anschließend ließ man abkühlen. Während des gesamten Herstellungsprozesses wurde stetig gerührt.

### Lösung 2:

466,8 g Kupfersulfat wurden in 2880 ml dest. H₂O bei Raumtemperatur gelöst.

Nach Abkühlung von Lösung 1 wurden die beiden Lösungen gemischt und 1 Stunde lang gerührt. Es entstand eine schwarze Lösung mit feinen weißen Partikeln.

Nach Verdüsen der Lösung / Suspension bei 380 °C im Pulsationsreaktor wurde ein Pulver mit folgender Charakteristik erhalten:

| | |
|---|---|
| Pulver: | grau/schwarz |
| BET: | 5 -7 m2/g |
| XRD (siehe Figur 1): | Zeigt Hauptpeak bei 22 |
| Kristallitgröße: | 207 Å |

### Beispiel 7 (erfindungsgemäß)

11820 ml dest. H₂O wurden in einem Gefäß vorgelegt. In dieses Gefäß wurde 3150,12 g Ammoniumheptamolybdat-tetrahydrat zugegeben, dann wurde mit dem Heizen begonnen und gewartet, bis sich alles vollständig gelöst hatte.

Danach erfolgte eine Zugabe von 822,24 g Ammoniummetawolframat und es wurde gewartet bis sich alles gelöst hatte. Jetzt wurde bis ca. 80 °C aufgeheizt, anschließend wurde 585,6 g Ammoniummetavanadat zugeben und weiter auf 100 °C erwärmt. Diese Temperatur wurde 5 Stunden gehalten, dann auf Raumtemperatur abgekühlt.

Nach Abkühlung auf Raumtemperatur wurden 444 g Ammoniumacetat zugegeben. Während des gesamten Herstellungsprozesses wurde stetig gerührt.

### Dotierung:

Der Ansatz wurde mit Eisen und Kupfer dotiert. Dazu wurden 3,72 g Kupferacetat und 8,28 g Eisennitrat in jeweils 240 ml dest. H₂O gelöst und anschließend in die Lösung gegeben.

Nach Verdüsen der Lösung bei 380°C im Pulsationsreaktor wurde ein Pulver mit folgender Charakteristik erhalten:

| | |
|---|---|
| Farbe des Pulvers: | grau/schwarz |
| BET: | 6 |
| XRD (siehe Figur 2): | Zeigt Hauptpeak bei 22 |
| Kristallitgröße: | 210 Å |

### Beispiel 8 (Erzeugung von beschichteten Katalysatoren)

Zur Durchführung der Beschichtung kam eine Wirbelbett-Coating-Vorrichtung zum Einsatz.

Die Beschichtung der Steatit-Kugeln mit den verschiedenen Mischoxid-Aktivmassen aus Beispiel 5 bis 7 erfolgte unter folgenden Bedingungen:

Es wurden 22,22 g des Pulvers in einen Messzylinder eingewogen, mit 500 ml dest. H₂O aufgeschlämmt. Die entstandene Suspension wurde intensiv gerührt. Anschließend wurde 8,89 g Binder zugegeben und 1 h lang auf einem Magnetrührer gerührt. Das "Coating" der hergestellten Suspension erfolgte auf einer Einwaage von 80 g Steatitkugeln mit (2 - 4 mm), wobei die Aktivmassenbeladung 20 % betrug (50 g Pulver per 200 g Steatitkugeln). Der Katalysator wurde dann bei 110 °C an Luft getrocknet.

### Beispiel 9 (Ermittlung der katalytischen Leistungsdaten der Katalysatoren)

In ein 120 cm langes Reaktionsrohr mit einem Innendurchmesser von 24,8 mm wurden 21 g Katalysator (d.h. die vorstehend erläuterten beschichteten Steatitkugeln), verdünnt mit 350 g Steatitkugeln mit Durchmesser 4.5 mm zur Vermeidung von Hotspots, auf einer Länge von 105 cm eingefüllt. Das Reaktionsrohr befand sich in einer flüssigen Salzschmelze, die auf Temperaturen bis 500 °C aufgeheizt werden konnte. In der Katalysatorschüttung befand sich ein 3 mm Schutzrohr mit eingebautem Thermoelement über das die Katalysatortemperatur über die komplette Katalysatorkombination angezeigt werden konnte.

Zur Ermittlung der katalytischen Leistungsdaten wurde Acrolein mittels eines Sättigers in die Gasphase gebracht. Der Sättiger wurde mit einem Luft-Inertgasgemisch so durchströmt und die Thermostattemperatur des Sättigers so eingestellt, dass sich ein Acrolein-Gehalt von 5 Vol.-% im Feedgas ergab. Der Reaktorfeed bestand aus einem Gemisch von 7 Vol.-% O, 10 Vol.-% Dampf und Rest N₂. Die Acrolein-Belastung war maximal 150 Nl/lh.

Der Acroleinumsatz und die Acrylsäureselektivität wurden bei einer mittleren Katalysatortemperatur von 250 °C bestimmt. Die Ergebnisse der Tests mit den Katalysatoren, die die Aktivmasse gemäß den in der Tabelle genannten Beispielen enthalten, sind in Tabelle 1 aufgeführt.

**Tabelle 1**

| | | **Beispiel 5 (Vergleichsbeispiel, Stand der Technik)** | **Beispiel 6 (erfindungsgemäß)** | **Beispiel 7 (erfindungsgemäß)** |
|---|---|---|---|---|
| **Acroleinumsatz** | | **98,3** | **99,2** | **98,7** |
| **Acrylsäureselektivität** | | **95,1** | **95,3** | **95,8** |
| **Acrylsäureausbeute** | | **93,5** | **94,5** | **94,6** |

Anhand der ermittelten Ergebnisse zeigt sich eine Verbesserung der erfindungsgemäßen Katalysatoren hinsichtlich Acroleinumsatz, Acrylsäureselektivität und Acrylsäureausbeute gegenüber einem Katalysator nach dem Stand der Technik.

## Patentansprüche

1. Verfahren zur Herstellung eines nanokristallinen Molybdänmischoxids umfassend die Schritte
a) des Einbringens einer Lösung, Suspension oder Aufschlämmung, welche eine Molybdänausgangsverbindung und wenigstens eine weitere metallhaltige Ausgangsverbindung, ausgewählt aus einer wolframhaltigen und/oder vanadiumhaltigen Ausgangsverbindung, enthält, in eine Reaktionskammer mittels eines Trägerfluids,
b) einer thermischen Behandlung der Lösung, Suspension oder Aufschlämmung, welche die Molybdänausgangsverbindung und die wenigstens eine weitere metallhaltige Ausgangsverbindung enthält, in einer Behandlungszone mittels einer pulsierenden Strömung bei einer Temperatur von 200 bis 500 °C,
c) des Bildens von nanokristallinem Molybdänmischoxid,
d) des Ausbringens des in Schritt b) und c) erhaltenen nanokristallinen Molybdänmischoxids aus dem Reaktor,
wobei die Molybdänausgangsverbindung ein Molybdat, die wolframhaltige Ausgangsverbindung ein Wolframat und die vanadiumhaltige Ausgangsverbindung ein Vanadat ist, und wobei zu der Lösung, Suspension, oder Aufschlämmung, welche die Molybdänausgangsverbindung und die wenigstens eine weitere metallhaltige Ausgangsverbindung enthält, vor dem oder während dem Einbringen in die Reaktionskammer eine Lösung, Suspension oder Aufschlämmung eines Kupfer und/oder Eisensalzes gegeben wird.

2. Verfahren nach Anspruch 1, **dadurch kennzeichnet, dass** die Molybdänausgangsverbindung Ammoniumheptamolybdat-tetrahydrat ist.

3. Verfahren nach Anspruch 1 oder 2, **dadurch gekennzeichnet, dass**, die wenigstens eine weitere metallhaltige Ausgangsverbindung Ammoniummetawolframat und/oder Ammoniummetavanadat ist.

4. Verfahren nach einem der Ansprüche 1 bis 3, **dadurch gekennzeichnet, dass** das Trägerfluid ein Gas ist.

5. Nanokristallines Molybdänmischoxid, erhältlich durch ein Verfahren nach einem der Ansprüche 1 bis 4 mit einer Kristallitgröße im Bereich von 10 nm bis 450 nm.

6. Nanokristallines Molybdänmischoxid nach Anspruch 5, **dadurch gekennzeichnet, dass** es eine BET-Oberfläche von 1 bis 10 m²/g aufweist.

7. Verwendung eines nanokristallinen Molybdänmischoxids nach einem der Ansprüche 5 oder 6 als Katalysator für chemische Umsetzungen.

8. Verwendung nach Anspruch 7 zur Umsetzung von Acrolein zu Acrylsäure.

9. Katalysator, enthaltend das nanokristalline Molybdänmischoxid nach einem der Ansprüche 5 oder 6.

10. Katalysator nach Anspruch 9, **dadurch gekennzeichnet, dass** das Molybdänmischoxid als Beschichtung auf einem Träger vorliegt.

11. Verfahren zur Umsetzung von Acrolein zu Acrylsäure, wobei ein Katalysator nach Anspruch 9 oder 10 verwendet wird.

## Claims

1. Process for preparing a nanocrystalline molybdenum mixed oxide, which comprises the steps
a) introduction of a solution, suspension or slurry containing a molybdenum starting compound and at least one further metal-containing starting compound selected from among a tungsten-containing starting compound and a vanadium-containing starting compound into a reaction chamber by means of a carrier fluid,
b) thermal treatment of the solution, suspension or slurry containing the molybdenum starting compound and the at least one further metal-containing starting compound in a treatment zone by means of pulsating flow at a temperature of from 200 to 500°C,
c) formation of nanocrystalline molybdenum mixed oxide,
d) discharge of the nanocrystalline molybdenum mixed oxide obtained in steps b) and c) from the reactor,
where the molybdenum starting compound is a molybdate, the tungsten-containing starting compound is a tungstate and the vanadium-containing starting compound is a vanadate and a solution, suspension or slurry of a copper and/or iron salt is added to the solution, suspension or slurry containing the molybdenum starting compound and the at least one further metal-containing starting compound before or during introduction into the reaction chamber.

2. Process according to Claim 1, **characterized in that** the molybdenum starting compound is ammonium heptamolybdate tetrahydrate.

3. Process according to Claim 1 or 2, **characterized in that** the at least one further metal-containing starting compound is ammonium metatungstate and/or ammonium metavanadate.

4. Process according to any of Claims 1 to 3, **characterized in that** the carrier fluid is a gas.

5. Nanocrystalline molybdenum mixed oxide obtainable by a process according to any of Claims 1 to 4 and having a crystallite size in the range from 10 nm to 450 nm.

6. Nanocrystalline molybdenum mixed oxide according to Claim 5, **characterized in that** it has a BET surface area of from 1 to 10 m²/g.

7. Use of a nanocrystalline molybdenum mixed oxide according to either Claim 5 or 6 as catalyst for chemical reactions.

8. Use according to Claim 7 for converting acrolein into acrylic acid.

9. Catalyst containing the nanocrystalline molybdenum mixed oxide according to either Claim 5 or 6.

10. Catalyst according to Claim 9, **characterized in that** the molybdenum mixed oxide is present as coating on a support.

11. Process for converting acrolein into acrylic acid, wherein a catalyst according to Claim 9 or 10 is used.

## Revendications

1. Procédé pour la préparation d'un oxyde mixte, nanocristallin, à base de molybdène, comprenant les étapes consistant à
a) introduire une solution, une suspension ou une suspension épaisse, contenant un composé de départ à base de molybdène et au moins un autre composé de départ contenant du métal, choisi parmi un composé de départ contenant du tungstène et/ou contenant du vanadium, dans une chambre de réaction au moyen d'un fluide support,
b) traiter thermiquement la solution, la suspension ou la suspension épaisse, qui contient le composé de départ à base de molybdène et ledit au moins un autre composé de départ contenant du métal, dans une zone de traitement au moyen d'un flux pulsé à une température de 200 à 500°C,
c) former un oxyde mixte, nanocristallin, de molybdène,
d) évacuer, du réacteur, l'oxyde mixte, nanocristallin, à base de molybdène obtenu dans l'étape b) et l'étape c),
le composé de départ à base de molybdène étant un molybdate, le composé de départ contenant du tungstène étant un tungsténate et le composé de départ contenant du vanadium étant un vanadate et une solution, une suspension ou une suspension épaisse d'un sel de cuivre et/ou de fer étant ajoutée à la solution, à la suspension ou à la suspension épaisse, qui contient le composé de départ à base de molybdène et ledit au moins un autre composé de départ contenant du métal, avant ou pendant l'introduction dans la chambre de réaction.

2. Procédé selon la revendication 1, **caractérisé en ce que** le composé de départ à base de molybdène est l'heptamolybdate d'ammonium tétrahydraté.

3. Procédé selon la revendication 1 ou 2, **caractérisé en ce que** ledit au moins un autre composé de départ contenant du métal est le métatungsténate d'ammonium et/ou le métavanadate d'ammonium.

4. Procédé selon l'une quelconque des revendications 1 à 3, **caractérisé en ce que** le fluide support est un gaz.

5. Oxyde mixte, nanocristallin, à base de molybdène, pouvant être obtenu par un procédé selon l'une quelconque des revendications 1 à 4, présentant une grosseur des cristaux dans la plage de 10 nm à 450 nm.

6. Oxyde mixte, nanocristallin, à base de molybdène selon la revendication 5, **caractérisé en ce qu'**il présente une surface BET de 1 à 10 m²/g.

7. Utilisation d'un oxyde mixte, nanocristallin, à base de molybdène selon l'une quelconque des revendications 5 ou 6 comme catalyseur pour des transformations chimiques.

8. Utilisation selon la revendication 7 pour la transformation d'acroléine en acide acrylique.

9. Catalyseur, contenant l'oxyde mixte, nanocristallin, à base de molybdène selon l'une quelconque des revendications 5 ou 6.

10. Catalyseur selon la revendication 9, **caractérisé en ce que** l'oxyde mixte à base de molybdène se trouve sous forme de revêtement sur un support.

11. Procédé pour la transformation d'acroléine en acide acrylique, un catalyseur selon la revendication 9 ou 10 étant utilisé.
